# EUROPEAN PATENT SPECIFICATION

(11) **EP 1 402 788 B1**
(45) Date of publication and mention of the grant of the patent: **31.12.2008**
(21) Application number: 02736016.3
(22) Date of filing: 06.06.2002
(51) Int. Cl.: A23L 1/305, A61K 31/198, A61K 31/205, A61P 3/02, A61P 43/00

(54) **BODY TEMPERATURE-RAISING AGENT OF AMINO ACIDS FOR EATING OR DRINKING AND FOR MEDICAL USE**
ESSBARE BZW. TRINKBARE SOWIE MEDIZINISCH VERWENDBARE, DIE KÖRPERTEMPERATUR ERHÖHENDE AMINOSÄUREZUSAMMENSETZUNG
AGENT D'ELEVATION DE LA TEMPERATURE CORPORELLE A BASE D'ACIDES AMINES DESTINE A ETRE MANGE OU BU ET DESTINE A UN USAGE MEDICAL

(30) Priority: 08.06.2001 JP 2001173983
(43) Date of publication of application: 31.03.2004
(73) Proprietor: RIKEN, Wako-shi, Saitama 351-0198 (JP); Abe, Takashi, Warabi-shi, Saitama 335-0003 (JP); Meiji Dairies Corporation, Koto-ku, Tokyo 136-8908 (JP)
(72) Inventor: ABE, Takashi, Warabi-shi, Saitama 335-0003 (JP); TSUNOO, Hajime, Oota-ku, Tokyo 145-0065 (JP); UCHIDA, Masayuki, Kouza-gun, Kanagawa 253-0111 (JP); WAKABAYASHI, Takae, Odawara-shi, Kanagawa 250-0874 (JP); TSUCHITA, Hiroshi, Nishitokyo-shi, Tokyo 202-0015 (JP)
(74) Representative: HOFFMANN EITLE
(86) International application number: PCT/JP2002/005584
(87) International publication number: WO 2002/100193

(56) References cited:
- EP-A- 0 917 826
- WO-A1-97/43912
- JP-A- 10 330 264
- JP-A- 11 253 130
- JP-A- 2000 228 967
- US-B1- 6 221 836
- DATABASE WPI Section Ch, Week 199409 Derwent Publications Ltd., London, GB; Class B05, AN 1994-071835 XP002051438 -& JP 06 024976 A (NIPPON STEEL CORP) 1 February 1994 (1994-02-01) & PATENT ABSTRACTS OF JAPAN vol. 018, no. 236 (C-1196), 6 May 1994 (1994-05-06) & JP 06 024976 A (RIKAGAKU KENKYUSHO; others: 01), 1 February 1994 (1994-02-01)
- G.J. PETERS ET AL.: "Effect of pyrimidine nucleosides om body temperature of man and rabbit in relation to pharmacokinetic data" PHARMACEUTICAL RESEARCH, vol. 4, no. 2, 1987, pages 113-119, XP008058917 USNEW YORK, NY

## Description

The present invention relates to amino acid mixture-containing agents capable of raising body temperature for food or drink use or medical use.

### Technical Field to which the Invention Belongs

The present invention relates to amino acid compositions and amino acid composition solutions made up of specified amino acid constituents. The amino acid composition and the solution thereof of the present invention have excellent physiological functions, particularly a body temperature raising function. Besides raising body temperature, it stimulates metabolism. Thus they are effective for improvement of basal metabolism, reduction of body fat accumulation and reduction of excess sensitivity to cold as well as being a means to warm a cold body in cold regions.

### Background Art

Conventionally, beverages have been known whose active components are capsaicins (JP-A-2000-189121) utilizing the function of the pungent spice component capsaicin to facilitate energy metabolism.

The use of an amino acid composition is presented in JP 06-024976, which is effective for the decrease of lipid in blood or liver, resulting from hepatic insufficiency caused by alcohol or cancer, or effective for the improvement of the lowered function of lactic acid metabolism. Further US 6,221,836 relates to an anabolic protein composition, which, apart from albumine protein, comprises mixtures of amino acids producing an effect in increasing the lean body mass or muscle tissue, increasing fat loss or decreasing the person's body fat and enhancing the protein concentration or muscle mass of a mammal.

### Problems that the Invention is to Solve

Above mentioned capsaicins are pungent compounds contained in peppers. The amount included in one dose has been limited since pungency is strongly felt when large amounts of capsaicins are added. In order to warm the body, a method in which hot food and drink are taken and heat is thus incorporated in the body is also feasible, but this effect is transient. Thus, as a composition having a function to improve energy metabolism, it is required that this effect is rapidly obtained and is sustained for some time thereafter. Also, an excellent taste and the like are necessary in the case of oral ingestion.

### Means for Solving the Problems

In order to achieve the above objects, the present inventors investigated large numbers of amino acid compositions which were seen to be promising after looking at this problem from various angles. A great number of amino acid compositions were prepared by changing types, combinations, and amounts of amino acids, and their physiological functions were studied in detail. As a result, the present inventors have found for the first time that an amino acid composition with a particular constitution raises basal body temperature and that this effect is sustained for a long time. As the result of further study, the present invention has been completed based on these useful findings.

### Brief Description of the Drawings

Fig. 1 is a graph showing the average rise in the basal body temperature at each time course when the amino acid composition of the present invention (16 kinds of amino acids in Table 1) was given to rats at each dose.
Fig. 2 is a graph showing the time courses of average basal body temperature when the amino acid composition of the present invention (16 kinds of amino acids in Table 1) was given to rats at each dose.
Fig. 3 is a graph showing the average maximum basal body temperature elevation when the amino acid composition of the present invention (17 kinds of amino acids in Table 4) was given to rats at each dose. Student's t-test was employed for statistical analysis of the experimental results.
Fig. 4 is a graph showing average rise in the basal body temperature at each time course when the amino acid composition of the present invention (17 kinds of amino acids in Table 4) was given to rats. Student's t-test was employed for statistical analysis of the experimental results.
Fig. 5 is a graph showing the time course of average basal body temperature when the amino acid composition of the present invention (17 kinds of amino acids in Table 4) was given to rats.

The present invention is described below.

The first amino acid mixture-containing composition of the present invention consists of threonine, proline, glycine, valine, isoleucine, leucine, tyrosine, phenylalanine, lysine, aspartic acid, serine, glutamic acid, alanine, methionine, histidine and arginine. These 16 amino acids as one mixture constitutes the basic active ingredient of the agent capable of raising body temperature of the present invention.

The second amino acid mixture-containing composition of the present invention consists of threonine, proline, glycine, valine, isoleucine, leucine tyrosine, phenylalanine, lysine, aspartic acid, serine, glutamic acid, alanine, methionine, histidine, arginine and carnitine. Carnitine is regarded here as one of amino acids.

The third amino acid mixture-containing composition which is not covered by the present invention consists of threonine, proline, glycine, valine, isoleucine, leucine, tyrosine, phenylalanine, lysine, aspartic acid, serine, glutamic acid, alanine, methionine, histidine, arginine and tryptophan. Although no protection is sought for the third amino acid mixture-containing composition as such, the present invention pertains to its use in food or drink or its medical use for raising body temperature.

The fourth amino acid mixture-containing composition of the present invention consists of threonine, proline, glycine, valine, isoleucine, leucine, tyrosine, phenylalanine, lysine, aspartic acid, serine, glutamic acid, alanine, methionine, histidine, arginine, tryptophan and carnitine.

The first amino acid mixture-containing composition of the present invention contains 16 kinds of amino acids as essential components. It is suitable that the respective amounts of each amino acid are in a certain ratio, as follows. It is preferred to have a ratio of from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 miles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine and from 0.1 to 5 moles of arginine.

The second amino acid mixture-containing composition of the present invention contains 17 kinds of amino acids as essential components. It is suitable that the respective amounts of each amino acid are in a certain ratio, as follows. It is preferred to have a ratio of from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 miles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine, from 0.1 to 5 moles of arginine and from 0.1 to 5 moles of carnitine.

The third amino acid mixture-containing composition which is not covered by the present invention contains 17 kinds of amino acids as essential components. It is suitable that the respective amounts of each amino acid are in a certain ratio, as follows. It is preferred to have a ratio of from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 miles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine, from 0.1 to 5 moles of arginine and from 0.1 to 5 moles of tryptophan.

The fourth amino acid mixture-containing composition of the present invention contains 18 kinds of amino acids as essential components. It is suitable that the respective amounts of each amino acid are in a certain ratio, as follows. It is preferred to have a ratio of from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 miles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine, from 0.1 to 5 moles of arginine, from 0.1 to 5 moles of tryptophan and from 0.1 to 5 moles of carnitine.

The amino acid mixture-containing agents capable of raising body temperature for food or drink use or medical use of the present invention are those in which one of the above first, second and fourth amino acid mixture-containing compositions is the active ingredient. Each amino acid may be free, or a pharmaceutically or food scientifically acceptable salt. Representative salts include hydrochloride and lactate, but these are only examples and the present invention is not limited to these salts. Amino acids each may be orally ingested as such in a mixed powder or an aqueous solution form. However, all of the amino acids have bad taste and are difficult to swallow in either liquid or solid form. Thus, it is better to make them tasty so as to be easily drunk or to make them all into a biscuit form to be easily eaten by using an additive acceptable for food or drink use or medical use together with the active ingredient (from the first to the fourth amino acid mixtures). For example, an easily drinkable preparation can be made by adding an acidifier such as citric acid and a sweetener such as sugar to the aqueous solution of the active ingredient. Also, if refined starch or the like is added to and mixed with the active ingredient in mixed powder form and the mixture is baked into a biscuit form, it can be easily eaten.

As mentioned above, the amino acid mixture-containing agents capable of raising body temperature for food or drink use or medical use of the present invention may be ingested as is in powder form, or dissolved to form an aqueous solution and then ingested. The ingestion method may be any of the common routes of administration such as oral administration, rectal administration, intravenous injection, and drip infusion

In the case of oral administration, they may be used as powder, granule, tablet, capsule, troche, and the like by mixing with a pharmaceutically acceptable carrier, excipient, or diluent in addition to making them easily drunk or eaten as foods. However, a long time is sometimes required for absorption of solid powder and tablet agents, and thus oral administration of the active ingredient itself is desirable. In this case, the active component may be administered in the aforementioned solution along with appropriate additives, for example, salts such as sodium chloride, a pH adjuster and a chelating agent. In the case of using as an injectable agent, it is better to use those in which an appropriate buffer or isotonic solution is added to the active ingredient, which is dissolved in sterile distilled water

The ingestion period is not particularly limited. It may be ingested at any suitable time, and is suitable for ingestion , for example, as a drink (including those for medical use in addition to ordinary types such as soft drinks and powder for drinks).

The dosage of the amino acid mixture-containing agents capable of raising body temperature for food or drink use or medical use of the present invention can be set within a wide range. The amount of the active ingredient, usually the amount of from 0.5 to 5 g per dose, preferably from 1 to 3 g per dose, and from 1 to 20 g per day, preferably from 3 to 10 g per day, is determined depending on the administration method or the objective of the use. In the case of administering a solution, from 10 to 1000 ml per dose of a solution 0.5 to 10% by weight, and preferably from 100 to 400 ml per dose of a solution of from 1 to 4% by weight is administered.

As is shown in the examples described below, the amino acid mixture-containing agents capable of raising body temperature for food or drink use or medical use of the present invention have excellent capacity to raise basal body temperature. In conjunction with this, they increase basal metabolism and daily energy consumption. Thus, they are effective for reduction of body fat accumulation and excess sensitivity to cold. Therefore, the present amino acid compositions can be made into various medicines such as body temperature raising agents, anti-cold sensitivity agents by formulating them for medical use.

The amino acid mixture-containing agents capable of raising body temperature for food or drink use or medical use of the present invention have highly effective body temperature raising function. They may be used in solution as mentioned above, particularly in aqueous solution, in addition the original powder form. In the case of the solution, the composition of the present invention may be dissolved as is in water to prepare the solution, or each amino acid may be separately dissolved in water to realize the aforementioned constitution in the solution.

The examples of the present invention are described below.

### Example 1

### (1) Method

Four groups of 8- to 9-weeks old Crj:CD(SD)IGS male rats with 4 rats per group were prepared, and were used for the experiments after overnight fasting.

The first group was control (saline). For the second group 0.2 g of the amino acid composition of the invention per 100 g of body weight (2 g/kg), 0.1 g per 100 g of body weight (1 g/kg) for the third group, and 0.05 g per 100 g of body weight (0.5 g/kg) for the fourth group were administered once intraperitoneally. The amino acid composition of the invention (the composition having 16 kinds of amino acids in the given constitution) was suspended in 0.8 ml of saline per 100 g of body weight (8 ml/kg). For the control group, 0.8 ml of saline (Hikari Seiyaku KK, Pharmacopoeia of Japan saline solution, Lot. 9911HC) per 100 g of body weight (8 ml/kg) alone was administered.

### (2) Substance to be tested

The amino acid composition shown in the following Table 1 is one embodiment of the composition of the invention. All amino acids were obtained from Wako Pure Chemical.

**(Table 1)**

| Amino acid | mole % |
|---|---|
| Histidine | 2.6 |
| Arginine | 3.6 |
| Isoleucine | 4.6 |
| Leucine | 6.3 |
| Lysine hydrochloride | 8.8 |
| Phenylalanine | 3.9 |
| Tyrosine | 6.1 |
| Valine | 6.0 |
| Aspartic acid | 0.2 |
| Serine | 2.5 |
| Glutamic acid | 3.3 |
| Proline | 18.4 |
| Glycine | 19.5 |
| Alanine | 6.3 |
| Threonine | 7.4 |
| Methionine | 0.5 |

### (3) Administration and measurement of body temperature

The substance to be tested was administered using an aliform needle for intravenous injection. In order to confirm that the drug was surely administered intraperitoneally, a dye was administered after the measured dose was given to confirm that the subject of testing was not administered into the intestine.

The animal was laid on the back under anesthesia with urethane (1.2 g/kg, i.p.), and the aliform needle for intravenous injection was kept in the peritoneal cavity for intraperitoneal administration. A probe for body temperature measurement was inserted from the anus into rectum and fixed. The body temperature was measured every 10 min. The temperature at 0 min after the administration was the basis. The basal body temperatures 10 min before, after 10 min, after 20 min, ... and so on up to after 120 min were measured, and the average value was calculated from the results of each time to make tables and figures (graphs).

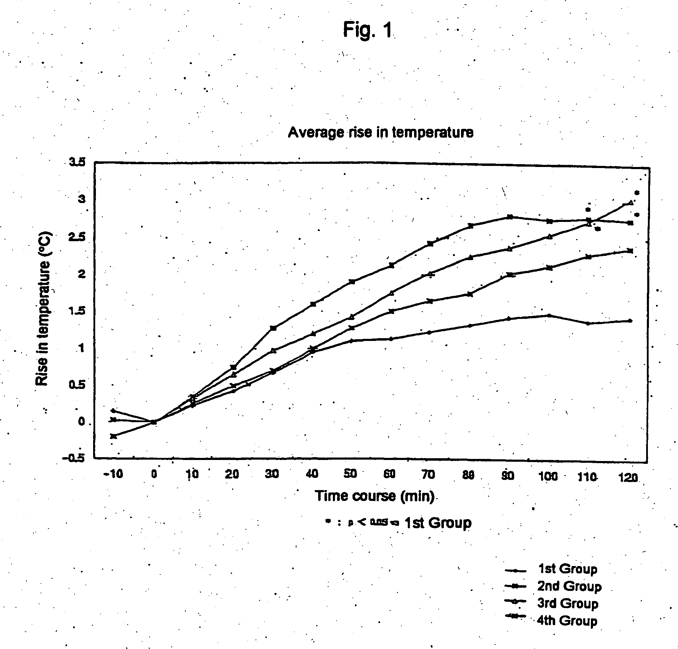

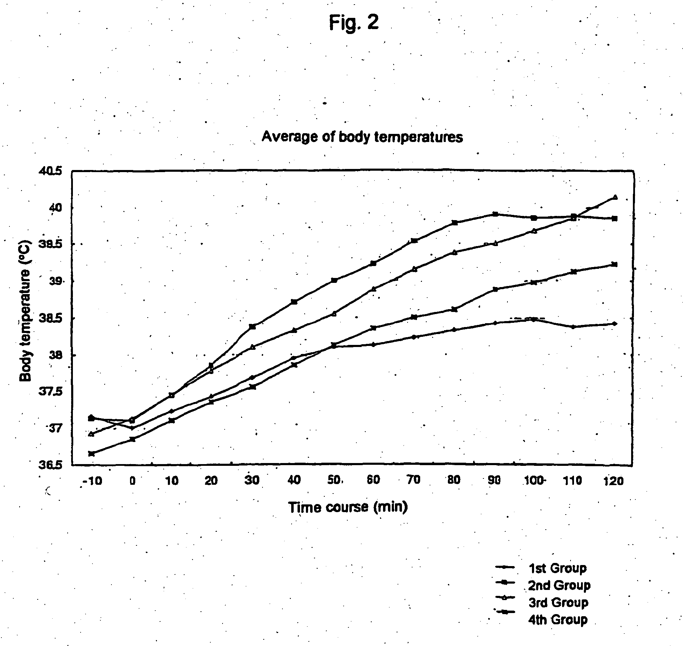

That is, the average value of the elevated temperature of the basal body temperature at each time course was shown in the following Table 2 and Fig. 1. The average of the basal body temperature at each time course is shown in the following Table 3 and Fig. 2.

**(Table 2)**

| Average of elevated temperature of basal body temperature at each time course | | | | | | | |
|---|---|---|---|---|---|---|---|
| Minute | -10 | 0 | 10 | 20 | 30 | 40 | 50 |
| 1st Group | 0.15 | 0 | 0.225 | 0.425 | 0.675 | 0.95 | 1.1 |
| 2nd Group | 0.025 | 0 | 0.35 | 0.75 | 1.275 | 1.6 | 1.9 |
| 3rd Group | -0.2 | 0 | 0.325 | 0.65 | 0.975 | 1.2 | 1.425 |
| 4th Group | -0.2 | 0 | 0.25 | 0.5 | 0.7 | 1 | 1.275 |
| | 60 | 70 | 80 | 90 | 100 | 110 | 120 |
| | 1.125 | 1.225 | 1.325 | 1.425 | 1.475 | 1.375 | 1.425 |
| | 2.125 | 2.425 | 2.675 | 2.8 | 2.75 | 2.775* | 2.75* |
| | 1.75 | 2.025 | 2.25 | 2.375 | 2.55 | 2.725* | 3.025* |
| | 1.5 | 1.65 | 1.75 | 2.025 | 2.125 | 2.275 | 2.375 |

| | | | | | | | |
|---|---|---|---|---|---|---|---|
| *p<0,05 versus 1st Group | | | | | | | |

**(Table 3)**

| Average of basal body temperature at each time course | | | | | | | |
|---|---|---|---|---|---|---|---|
| Minute | -10 | 0 | 10 | 20 | 30 | 40 | 50 |
| 1st Group | 37.15 | 37 | 37.225 | 37.425 | 37.675 | 37.95 | 38.1 |
| 2nd Group | 37.125 | 37.1 | 37.45 | 37.85 | 38.375 | 38.7 | 39 |
| 3rd Group | 36.925 | 37.125 | 37.45 | 37.775 | 38.1 | 38.325 | 38.55 |
| 4th Group | 36.65 | 36.85 | 37.1 | 37.35 | 37.55 | 37.85 | 38.125 |
| | 60 | 70 | 80 | 90 | 100 | 110 | 120 |
| | 38.125 | 38.225 | 38.325 | 38.425 | 38.475 | 38.375 | 38.425 |
| | 39.225 | 39.525 | 39.775 | 39.9 | 39.85 | 39.875 | 39.85 |
| | 38.875 | 39.15 | 39.375 | 39.5 | 39.675 | 39.85 | 40.15 |
| | 38.35 | 38.5 | 38.6 | 38.875 | 38.975 | 39.125 | 39.225 |

### (4) Results and discussion

As is clearly shown in the above results, the levels of the body temperature in the control group were slightly increased. The dose-dependent elevation of the body temperature was demonstrated in the groups given the substance to be tested. A significant difference was observed in the 2nd and 3rd groups compared to the control group.

### Example 2

### (1) Method

Two groups of 8- to 10-weeks old SD male rats and six per group were prepared. After overnight fasting, they were used for the experiments. The grouping was carried out depending on the body weight on the same day.

### (2) Substance to be tested

As the substance to be tested,17 kinds of amino acids (VAAM)-shown in Table 4 were used. The formulated ratios were as is shown in Table 4. All amino acids were obtained from Wako Pure Chemicals Co., Ltd

**(Table 4)**

| Seventeen kinds of amino acids: VAAM | | |
|---|---|---|
| Amino acid | Component weight (%) | mole (%) |
| Histidine | 3.2 | 2.6 |
| Arginine | 4.9 | 3.5 |
| Isoleucine | 4.7 | 4.5 |
| Leucin, | 6.4 | 6.1 |
| Lysine hydrochloride | 12.5 | 8.6 |
| Phenylalanine | 5.0 | 3.8 |
| Tyrosine | 8.6 | 6.0 |
| Valine | 5.5 | 5.9 |
| Aspartic acid | 0.2 | 0.2 |
| Serine | 2.1 | 2.5 |
| Glutamic acid | 3.7 | 3.2 |
| Proline | 16.5 | 18.0 |
| Glycine | 11.4 | 19.1 |
| Alanine | 4.3 | 6.1 |
| Threonine | 6.8 | 7.2 |
| Methionine | 0.6 | 0.5 |
| Tryptophan | 3.6 | 2.2 |

### (3) Dosage

The first group was the control (injectable water). The rats in the second group were once administered orally 0.5 g/100 g body weight (5 g/kg) of 17 kinds of amino acids.

The substance to be tested of the amino acids was suspended in 1 ml/100 g body weight (10 ml/kg) of saline. The control animals were given 1 ml/100 g body weight (10 ml/kg) of saline solution (Hikari Seiyaku KK, Pharmacopoeia of Japan saline solution, Lot. 9911HC).

### (4) Temperature measurement

The animals were placed and positioned in Bollman cages. The probe for measuring the body temperature was inserted from the anus into rectum and fixed. The body temperature was measured every 10 to 15 min. When the levels of the body temperature became stable, the substance to be tested was administered. Then, using the temperature at 0 min after the ingestion as the standard, the body temperature was measured every 10 min until 90 min after the ingestion. The average values were calculated from the results in each group and shown in the graphs. The maximally elevated body temperatures were averaged in each group, and the differences between groups were shown.

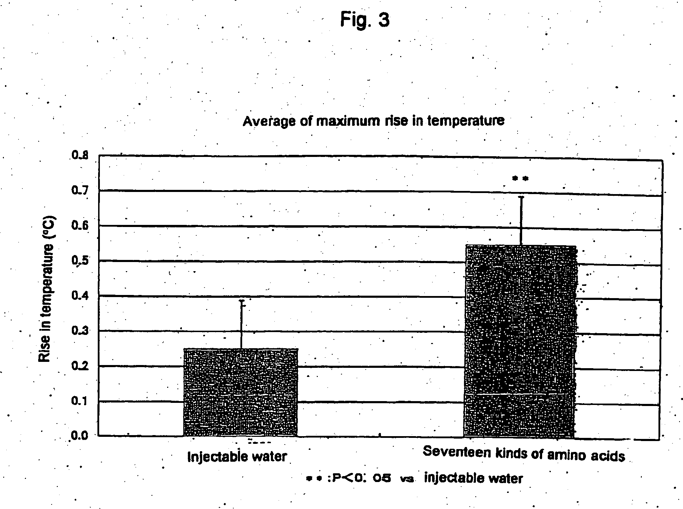

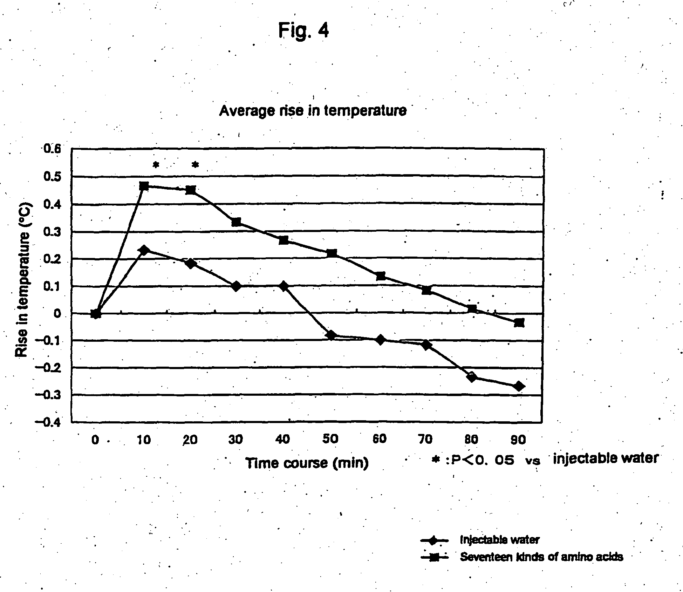

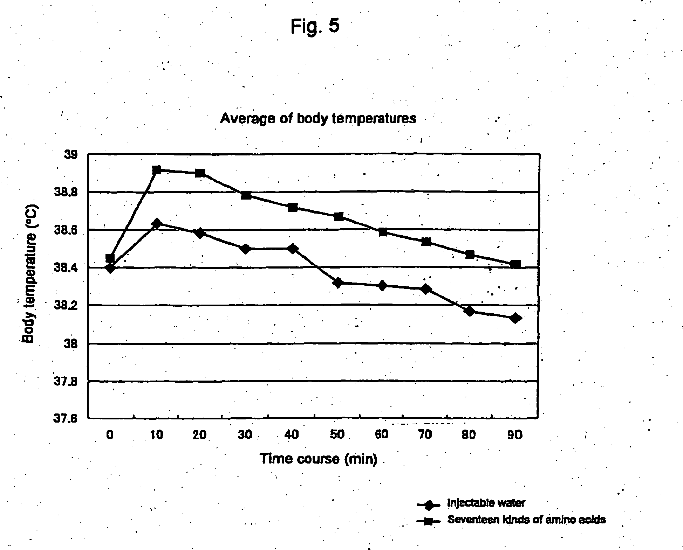

For 17 kinds of amino acids, the average values of the maximally elevated body temperatures were shown in Fig. 3, and the average values of the increment of the body temperatures and body temperatures per se at each time course are shown in Figs. 4 and 5, respectively.

### (5) Results and discussion

As is clearly shown in the above results, the levels of the temperature in the control group were slightly increased over the time course. The animals in the group given the substance to be tested (17 kinds of amino acids: VAAM) exhibited the excellent elevation of the body temperature.

### Effect of the Intention

The present invention has found that an active ingredient made up of certain amino acids has a novel use - an excellent action for elevation of body temperature. By administering the amino acid mixture-containing agents of the invention capable of raising body temperature for food or drink use or medical use, everyday body temperature is elevated, resulting in increased energy consumption and high basal metabolism. They are also effective for the elevation in basal body temperature, the reduction in body fat accumulation and alleviation of excess sensitivity to cold, as well as for warming the body when it is cold.

## Claims

1. An amino acid mixture-containing agent capable of raising body temperature for food or drink use or medical use, which comprises, as an active ingredient, an amino acid mixture consisting of threonine, proline, glycine, valine, isoleucine, leucine, tyrosine, phenylalanine, lysine, aspartic acid, serine, glutamic acid, alanine, methionine, histidine and arginine and optionally in addition carnitine or tryptophane and carnitine.

2. An amino acid mixture-containing agent according to claim 1, wherein the ratio of the amino acids is from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 moles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine and from 0.1 to 5 moles of arginine.

3. An amino acid mixture-containing agent according to claim 1, wherein the ratio of the amino acids is from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 moles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine, from 0.1 to 5 moles of arginine and from 0.1 to 5 moles of carnitine.

4. An amino acid mixture-containing agent according to claim 1, wherein the ratio of the amino acids is from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 moles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine, from 0.1 to 5 moles of arginine, from 0.1 to 5 moles of tryptophan and from 0.1 to 5 moles of carnitine.

5. An amino acid mixture-containing agent according to any of claims 1 to 4, which consists of the active ingredient according to any of claims 1 to 4 and an additive acceptable for food or drink use or medical use.

6. Use of an amino acid mixture-containing agent comprising, as an active ingredient, an amino acid mixture consisting of threonine, proline, glycine, valine, isoleucine, leucine, tyrosine, phenylalanine, lysine, aspartic acid, serine, glutamic acid, alanine, methionine, histidine, arginine and tryptophane for raising body temperature for food or drink use.

7. Use of an amino acid mixture-containing agent according to claim 6, wherein the ratio of the amino acids is from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 moles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine, from 0.1 to 5 moles of arginine and from 0.1 to 5 moles of tryptophan.

8. Use of an amino acid mixture-containing agent according to claim 6 or 7, which consists of the active ingredient as defined in claim 6 or 7 and an additive acceptable for food or drink use.

9. Use of an amino acid mixture consisting of threonine, proline, glycine, valine, isoleucine, leucine, tyrosine, phenylalanine, lysine, aspartic acid, seine, glutamic acid, alanine, methionine, histidine, arginine and tryptophane for preparing an agent raising body temperature in medical use.

10. Use of an amino acid mixture, according to claim 9, wherein the ratio of the amino acids is from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 moles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine, from 0.1 to 5 moles of arginine and from 0.1 to 5 moles of Tryptophan.

11. Use according to claim 9 or 10, wherein the agent raising body temperature consists of the amino acid mixture as defined in claim 9 or 10and an additive acceptable for medical use.

12. An amino acid mixture consisting of threonine, proline, glycine, valine, isoleucine, leucine, tyrosine, phenylalanine, lysine, aspartic acid, serine, glutamic acid, alanine, methionine, histidine, arginine and tryptophan for medical use in a method for raising body temperature.

13. The amino acid mixture according to claim 12, wherein the ratio of the amino acids is from 2 to 15 moles of threonine, from 4 to 30 moles of proline, from 7 to 20 moles of glycine, from 4 to 8 moles of valine, from 3 to 9 moles of isoleucine, from 2 to 12 moles of leucine, from 1 to 9 moles of tyrosine, from 0.5 to 5 moles of phenylalanine, from 5 to 11 moles of lysine, from 0.1 to 5 moles of aspartic acid, from 0.1 to 5 moles of serine, from 0.1 to 4 moles of glutamic acid, from 0.1 to 12 moles of alanine, from 0.1 to 5 moles of methionine, from 0.1 to 5 moles of histidine, from 0.1 to 5 moles of arginine and from 0.1 to 5 moles of tryptophan.

14. The amino acid mixture according to claim 12 or 13, wherein the agent raising body temperature consists of the amino acid mixture as defined in claim 12 or 13 and an additive acceptable for medical use.

## Patentansprüche

1. Aminosäuremischung-haltiges Mittel zur Verwendung in Eßwaren oder Getränken oder zur medizinischen Verwendung, das zur Erhöhung der Körpertemperatur geeignet ist und als Wirkstoff eine Aminosäuremischung bestehend aus Threonin, Prolin, Glycin, Valin, Isoleucin, Leucin, Tyrosin, Phenylalanin, Lysin, Asparaginsäure, Serin, Glutaminsäure, Alanin, Methionin, Histidin und Arginin und gegebenenfalls zusätzlich Carnitin oder Tryptophan und Carnitin umfaßt.

2. Aminosäuremischung-haltiges Mittel gemäß Anspruch 1, worin der Anteil der Aminosäuren 2 bis 15 mol Threonin, 4 bis 30 mol Prolin, 7 bis 20 mol Glycin, 4 bis 8 mol Valin, 3 bis 9 mol Isoleucin, 2 bis 12 mol Leucin, 1 bis 9 mol Tyrosin, 0,5 bis 5 mol Phenylalanin, 5 bis 11 mol Lysin, 0,1 bis 5 mol Asparaginsäure, 0,1 bis 5 mol Serin, 0,1 bis 4 mol Glutaminsäure, 0,1 bis 12 mol Alanin, 0,1 bis 5 mol Methionin, 0,1 bis 5 mol Histidin und 0,1 bis 5 mol Arginin beträgt.

3. Aminosäuremischung-haltiges Mittel gemäß Anspruch 1, worin der Anteil der Aminosäuren 2 bis 15 mol Threonin, 4 bis 30 mol Prolin, 7 bis 20 mol Glycin, 4 bis 8 mol Valin, 3 bis 9 mol Isoleucin, 2 bis 12 mol Leucin, 1 bis 9 mol Tyrosin, 0,5 bis 5 mol Phenylalanin, 5 bis 11 mol Lysin, 0,1 bis 5 mol Asparaginsäure, 0,1 bis 5 mol Serin, 0,1 bis 4 mol Glutaminsäure, 0,1 bis 12 mol Alanin, 0,1 bis 5 mol Methionin, 0,1 bis 5 mol Histidin, 0,1 bis 5 mol Arginin und 0,1 bis 5 mol Carnitin beträgt.

4. Aminosäuremischung-haltiges Mittel gemäß Anspruch 1, worin der Anteil der Aminosäuren 2 bis 15 mol Threonin, 4 bis 30 mol Prolin, 7 bis 20 mol Glycin, 4 bis 8 mol Valin, 3 bis 9 mol Isoleucin, 2 bis 12 mol Leucin, 1 bis 9 mol Tyrosin, 0,5 bis 5 mol Phenylalanin, 5 bis 11 mol Lysin, 0,1 bis 5 mol Asparaginsäure, 0,1 bis 5 mol Serin, 0,1 bis 4 mol Glutaminsäure, 0,1 bis 12 mol Alanin, 0,1 bis 5 mol Methionin, 0,1 bis 5 mol Histidin, 0,1 bis 5 mol Arginin, 0,1 bis 5 mol Tryptophan und 0,1 bis 5 mol Carnitin beträgt.

5. Aminosäuremischung-haltiges Mittel gemäß mindestens einem der Ansprüche 1 bis 4, das aus dem Wirkstoff gemäß mindestens einem der Ansprüche 1 bis 4 und einem zur Verwendung in Eßwaren oder Getränken oder zur medizinischen Verwendung akzeptablen Additiv besteht.

6. Verwendung eines Aminosäuremischung-haltigen Mittels, umfassend als Wirkstoff eine Aminosäuremischung, die aus Threonin, Prolin, Glycin, Valin, Isoleucin, Leucin, Tyrosin, Phenylalanin, Lysin, Asparaginsäure, Serin, Glutaminsäure, Alanin, Methionin, Histidin, Arginin und Tryptophan besteht, zur Erhöhung der Körpertemperatur zur Verwendung in Eßwaren oder Getränken.

7. Verwendung eines Aminosäuremischung-haltigen Mittels gemäß Anspruch 6, worin der Anteil der Aminosäuren 2 bis 15 mol Threonin, 4 bis 30 mol Prolin, 7 bis 20 mol Glycin, 4 bis 8 mol Valin, 3 bis 9 mol Isoleucin, 2 bis 12 mol Leucin, 1 bis 9 mol Tyrosin, 0,5 bis 5 mol Phenylalanin, 5 bis 11 mol Lysin, 0,1 bis 5 mol Asparaginsäure, 0,1 bis 5 mol Serin, 0,1 bis 4 mol Glutaminsäure, 0,1 bis 12 mol Alanin, 0,1 bis 5 mol Methionin, 0,1 bis 5 mol Histidin, 0,1 bis 5 mol Arginin und 0,1 bis 5 mol Tryptophan beträgt.

8. Verwendung eines Aminosäuremischung-haltigen Mittels gemäß Anspruch 6 oder 7, das aus dem Wirkstoff wie in Anspruch 6 oder 7 definiert und einem zur Verwendung in Eßwaren oder Getränken akzeptablen Additiv besteht.

9. Verwendung einer Aminosäuremischung, die aus Threonin, Prolin, Glycin, Valin, Isoleucin, Leucin, Tyrosin, Phenylalanin, Lysin, Asparaginsäure, Serin, Glutaminsäure, Alanin, Methionin, Histidin, Arginin und Tryptophan besteht, zur Herstellung eines die Körpertemperatur erhöhenden Mittels zur medizinischen Verwendung.

10. Verwendung einer Aminosäuremischung gemäß Anspruch 9, worin der Anteil der Aminosäuren 2 bis 15 mol Threonin, 4 bis 30 mol Prolin, 7 bis 20 mol Glycin, 4 bis 8 mol Valin, 3 bis 9 mol Isoleucin, 2 bis 12 mol Leucin, 1 bis 9 mol Tyrosin, 0,5 bis 5 mol Phenylalanin, 5 bis 11 mol Lysin, 0,1 bis 5 mol Asparaginsäure, 0,1 bis 5 mol Serin, 0,1 bis 4 mol Glutaminsäure, 0,1 bis 12 mol Alanin, 0,1 bis 5 mol Methionin, 0,1 bis 5 mol Histidin, 0,1 bis 5 mol Arginin und 0,1 bis 5 mol Tryptophan beträgt.

11. Verwendung gemäß Anspruch 9 oder 10, worin das die Körpertemperatur erhöhende Mittel aus der Aminosäuremischung wie in Anspruch 9 oder 10 definiert und einem zur medizinischen Verwendung akzeptablen Additiv besteht.

12. Aminosäuremischung, die aus Threonin, Prolin, Glycin, Valin, Isoleucin, Leucin, Tyrosin, Phenylalanin, Lysin, Asparaginsäure, Serin, Glutaminsäure, Alanin, Methionin, Histidin, Arginin und Tryptophan besteht, zur medizinischen Verwendung in einem Verfahren zur Erhöhung der Körpertemperatur.

13. Aminosäuremischung gemäß Anspruch 12, worin der Anteil der Aminosäuren 2 bis 15 mol Threonin, 4 bis 30 mol Prolin, 7 bis 20 mol Glycin, 4 bis 8 mol Valin, 3 bis 9 mol Isoleucin, 2 bis 12 mol Leucin, 1 bis 9 mol Tyrosin, 0,5 bis 5 mol Phenylalanin, 5 bis 11 mol Lysin, 0,1 bis 5 mol Asparaginsäure, 0,1 bis 5 mol Serin, 0,1 bis 4 mol Glutaminsäure, 0,1 bis 12 mol Alanin, 0,1 bis 5 mol Methionin, 0,1 bis 5 mol Histidin, 0,1 bis 5 mol Arginin und 0,1 bis 5 mol Tryptophan beträgt.

14. Aminosäuremischung gemäß Anspruch 12 oder 13, worin das die Körpertemperatur erhöhende Mittel aus der Aminosäuremischung, wie in Anspruch 12 oder 13 definiert, und einem zur medizinischen Verwendung akzeptablen Additiv besteht.

## Revendications

1. Agent contenant un mélange d'acides aminés capable d'élever la température corporelle pour usage alimentaire ou en boisson ou usage médical, lequel comprend, comme principe actif, un mélange d'acides aminés constitué de thréonine, proline, glycine, valine, isoleucine, leucine, tyrosine, phénylalanine, lysine, acide aspartique, sérine, acide glutamique, alanine, méthionine, histidine et arginine et facultativement en outre carnitine ou tryptophane et carnitine.

2. Agent contenant un mélange d'acides aminés selon la revendication 1, où le taux des acides aminés est de 2 à 15 moles de thréonine, de 4 à 30 moles de proline, de 7 à 20 moles de glycine, de 4 à 8 moles de valine, de 3 à 9 moles d'isoleucine, de 2 à 12 moles de leucine, de 1 à 9 moles de tyrosine, de 0,5 à 5 moles de phénylalanine, de 5 à 11 moles de lysine, de 0,1 à 5 moles d'acide aspartique, de 0,1 à 5 moles de sérine, de 0,1 à 4 moles d'acide glutamique, de 0,1 à 12 moles d'alanine, de 0,1 à 5 moles de méthionine, de 0,1 à 5 moles d'histidine et de 0,1 à 5 moles d'arginine.

3. Agent contenant un mélange d'acides aminés selon la revendication 1, où le taux des acides aminés est de 2 à 15 moles de thréonine, de 4 à 30 moles de proline, de 7 à 20 moles de glycine, de 4 à 8 moles de valine, de 3 à 9 moles d'isoleucine, de 2 à 12 moles de leucine, de 1 à 9 moles de tyrosine, de 0,5 à 5 moles de phénylalanine, de 5 à 11 moles de lysine, de 0,1 à 5 moles d'acide aspartique, de 0,1 à 5 moles de sérine, de 0,1 à 4 moles d'acide glutamique, de 0,1 à 12 moles d'alanine, de 0,1 à 5 moles de méthionine, de 0,1 à 5 moles d'histidine, de 0,1 à 5 moles d'arginine et de 0,1 à 5 moles de carnitine.

4. Agent contenant un mélange d'acides aminés selon la revendication 1, où le taux des acides aminés est de 2 à 15 moles de thréonine, de 4 à 30 moles de proline, de 7 à 20 moles de glycine, de 4 à 8 moles de valine, de 3 à 9 moles d'isoleucine, de 2 à 12 moles de leucine, de 1 à 9 moles de tyrosine, de 0,5 à 5 moles de phénylalanine, de 5 à 11 moles de lysine, de 0,1 à 5 moles d'acide aspartique, de 0,1 à 5 moles de sérine, de 0,1 à 4 moles d'acide glutamique, de 0,1 à 12 moles d'alanine, de 0,1 à 5 moles de méthionine, de 0,1 à 5 moles d'histidine, de 0,1 à 5 moles d'arginine, de 0,1 à 5 moles de tryptophane et de 0,1 à 5 moles de carnitine.

5. Agent contenant un mélange d'acides aminés selon l'une quelconque des revendications 1 à 4, lequel est constitué du principe actif selon l'une quelconque des revendications 1 à 4 et d'un additif acceptable pour usage alimentaire ou en boisson ou pour usage médical.

6. Utilisation d'un agent contenant un mélange d'acides aminés comprenant comme principe actif un mélange d'acides aminés constitué de thréonine, proline, glycine, valine, isoleucine, leucine, tyrosine, phénylalanine, lysine, acide aspartique, sérine, acide glutamique, alanine, méthionine, histidine, arginine et tryptophane pour élever la température corporelle pour usage alimentaire ou en boisson.

7. Utilisation d'un agent contenant un mélange d'acides aminés selon la revendication 6, où le taux des acides aminés est de 2 à 15 moles de thréonine, de 4 à 30 moles de proline, de 7 à 20 moles de glycine, de 4 à 8 moles de valine, de 3 à 9 moles d'isoleucine, de 2 à 12 moles de leucine, de 1 à 9 moles de tyrosine, de 0,5 à 5 moles de phénylalanine, de 5 à 11 moles de lysine, de 0,1 à 5 moles d'acide aspartique, de 0,1 à 5 moles de sérine, de 0,1 à 4 moles d'acide glutamique, de 0,1 à 12 moles d'alanine, de 0,1 à 5 moles de méthionine, de 0,1 à 5 moles d'histidine, de 0,1 à 5 moles d'arginine et de 0,1 à 5 moles de tryptophane.

8. Utilisation d'un agent contenant un mélange d'acides aminés selon la revendication 6 ou 7, lequel est constitué du principe actif selon la revendication 6 ou 7 et d'un additif acceptable pour usage alimentaire ou en boisson.

9. Utilisation d'un mélange d'acides aminés constitué de thréonine, proline, glycine, valine, isoleucine, leucine, tyrosine, phénylalanine, lysine, acide aspartique, sérine, acide glutamique, alanine, méthionine, histidine, arginine et tryptophane pour préparer un agent élevant la température corporelle en usage médical.

10. Utilisation d'un mélange d'acides aminés selon la revendication 9, où le taux des acides aminés est de 2 à 15 moles de thréonine, de 4 à 30 moles de proline, de 7 à 20 moles de glycine, de 4 à 8 moles de valine, de 3 à 9 moles d'isoleucine, de 2 à 12 moles de leucine, de 1 à 9 moles de tyrosine, de 0,5 à 5 moles de phénylalanine, de 5 à 11 moles de lysine, de 0,1 à 5 moles d'acide aspartique, de 0,1 à 5 moles de sérine, de 0,1 à 4 moles d'acide glutamique, de 0,1 à 12 moles d'alanine, de 0,1 à 5 moles de méthionine, de 0,1 à 5 moles d'histidine, de 0,1 à 5 moles d'arginine et de 0,1 à 5 moles de tryptophane.

11. Utilisation selon la revendication 9 ou 10, où l'agent élevant la température corporelle est constitué d'un mélange d'acides aminés selon la revendication 9 ou 10 et d'un additif acceptable pour usage médical.

12. Mélanges d'acides aminés constitué de thréonine, proline, glycine, valine, isoleucine, leucine, tyrosine, phénylalanine, lysine, acide aspartique, sérine, acide glutamique, alanine, méthionine, histidine, arginine et tryptophane pour usage médical dans un procédé pour élever la température corporelle.

13. Mélange d'acides aminés selon la revendication 12, où le taux des acides aminés est de 2 à 15 moles de thréonine, de 4 à 30 moles de proline, de 7 à 20 moles de glycine, de 4 à 8 moles de valine, de 3 à 9 moles d'isoleucine, de 2 à 12 moles de leucine, de 1 à 9 moles de tyrosine, de 0,5 à 5 moles de phénylalanine, de 5 à 11 moles de lysine, de 0,1 à 5 moles d'acide aspartique, de 0,1 à 5 moles de sérine, de 0,1 à 4 moles d'acide glutamique, de 0,1 à 12 moles d'alanine, de 0,1 à 5 moles de méthionine, de 0,1 à 5 moles d'histidine, de 0,1 à 5 moles d'arginine et de 0,1 à 5 moles de tryptophane.

14. Mélange d'acides aminés selon la revendication 12 ou 13, où l'agent élevant la température corporelle est constitué du mélange d'acides aminés selon la revendication 12 ou 13 et d'un additif acceptable pour usage médical.
